# EUROPEAN PATENT APPLICATION

(11) **EP 3 821 832 A1**
(43) Date of publication of application: **19.05.2021**
(21) Application number: 20207363.1
(22) Date of filing: 13.11.2020
(51) Int. Cl.: A61B 17/29, A61B 17/00

(54) **SURGICAL GRASPER**

(30) Priority: 15.11.2019 US 201962935925 P; 02.04.2020 US 202063004004 P; 17.09.2020 US 202017024075
(71) Applicant: Covidien LP, Mansfield, MA 02048 (US)
(72) Inventor: HOLSTEN, Henry E., Hamden, CT 06518 (US)
(74) Representative: Maschio, Antonio

(57) **Abstract**

A surgical grasper includes parallel jaws operatively coupled to an ergonomic handle assembly. The ergonomic handle assembly is configured to enable a clinician to hold the ergonomic handle assembly in a desired orientation most comfortable to the clinician to reduce fatigue during a surgical procedure without affecting the function of the parallel jaws.

## Description

### CROSS-REFERENCE TO RELATED APPLICATIONS

This application claims the benefit of and priority to U.S. Provisional Patent Application Nos. 62/935,925, filed on November 15, 2019, and 63/004,004, filed on April 2, 2020, the entire contents of each of which are incorporated by reference herein.

### FIELD

The disclosure relates to surgical devices for performing endoscopic surgical procedures. More specifically, the disclosure relates to a surgical grasper having parallel jaws operatively coupled to an ergonomic handle assembly.

### BACKGROUND

In minimally invasive surgery, the surgical procedure is performed by access to the surgical site through one or more small incisions. The surgical site is visualized by an endoscope inserted through one of the incisions and various surgical devices are inserted through the incisions to manipulate the tissue as desired. The advantages of minimally invasive surgery are well established which include reduced trauma to tissue, reduced chance of infection, faster patient recovery time, and lower hospital costs.

Minimally invasive surgical procedures typically require the tissue to be grasped and held or manipulated. It would be advantageous to provide a tissue grasper that applies uniform tissue compression and that is easy to use by a clinician.

### SUMMARY

In accordance with the disclosure, a surgical grasper includes a tool assembly, an elongate shaft, an actuation member, and a handle assembly. The tool assembly includes first and second jaws transitionable between an approximated configuration, in which, the first and jaws are adjacent each other to grasp tissue therebetween and a spaced apart configuration, in which, the first and second jaws are spaced apart from each other. The elongate shaft supports the tool assembly at a distal portion of the elongate shaft. The actuation member extends through the elongate shaft. The actuation member is operatively coupled to the tool assembly such that axial displacement of the actuation member causes transition of the first and second jaws between the approximated and spaced apart configurations. The handle assembly has an arcuate body. The arcuate body includes an actuation button and a gimbal assembly. The actuation button is operatively coupled to the actuation member to transition the first and second jaws between the approximated and spaced apart configurations. The gimbal assembly is configured to support the elongate shaft and the actuation member for off-axis movement and rotation.

In an aspect, the first and second jaws may be biased towards the spaced apart configuration.

In another aspect, the arcuate body of the handle assembly may have a diameter in the range from about 1 inch and about 4 inches.

In another aspect, the arcuate body of the handle assembly may have a diameter of about 2.4 inches.

In another aspect, the gimbal assembly may be configured to maintain the configuration of the first and second jaws during the off-axis movement and rotation of the handle assembly relative to the elongate shaft.

In yet another aspect, the arcuate body may be configured for off-axis movement less than about 45 degrees with respect to the elongate shaft.

In still yet another aspect, the arcuate body may define an aperture configured to receive the elongate shaft and the actuation member therethrough.

In still yet another aspect, the aperture of the arcuate body may have a conical shape.

In an aspect, the arcuate body may include a skirt configured to provide a fluid tight seal against the elongate shaft.

In an aspect, the arcuate body may be spherical.

In another aspect, the actuation member may be at least partially flexible.

In accordance with another aspect of the disclosure, a surgical grasper includes a tool assembly, an elongate shaft, an actuation member, and a handle assembly. The tool assembly includes first and second jaws and first, second, third, and fourth linkage members. The first and second jaws are transitionable between an approximated configuration, in which, the first and jaws are adjacent each other to grasp tissue therebetween and a spaced apart configuration, in which, the first and second jaws are spaced apart from each other. The first and second linkage members are coupled to each other about a first pivot. The first and second linkage members are coupled to the respective first and second jaws and the respective third and fourth linkage members about respective second and third pivots. The third and fourth linkage members are pivotably coupled to each other about a fourth pivot. The elongate shaft supports the tool assembly at a distal portion of the elongate shaft. The actuation member extends through the elongate shaft and operatively coupled to the tool assembly such that axial displacement of the actuation member causes transition of the first and second jaws between the approximated and spaced apart configurations. The handle assembly includes an actuation button and a gimbal assembly. The actuation button is operatively coupled to the actuation member to transition the first and second jaws between the approximated and spaced apart configurations. The gimbal assembly is configured to support the elongate shaft and the actuation member for off-axis movement and rotation.

In an aspect, the second and third pivots may be movable along the respective first and second jaws.

In another aspect, the fourth pivot may be coupled to the actuation member for concomitant axial displacement therewith.

In yet another aspect, the fourth pivot may be movable along a groove defined in the elongate shaft.

In still yet another aspect, the first and second jaws may include respective tissue contacting surfaces.

In still yet another aspect, each tissue contacting surface may have a convex profile.

In still yet another aspect, the second and third pivots may be axially movable along a length of the respective first and second jaws.

In an aspect, the gimbal assembly may be configured to maintain the configuration of the first and second jaws during movement of the handle assembly relative to the elongate shaft.

In another aspect, the first and second linkage members may be pivotably coupled to the respective first and second jaws.

In accordance with yet another aspect of the disclosure, a surgical grasper includes a tool assembly including first and second jaws transitionable between approximated and spaced apart configurations, an actuation member operatively coupled to the tool assembly to transition the first and second jaws between the approximated and spaced apart configurations, and an arcuate body. The arcuate body includes an actuation button operatively coupled to the actuation member to transition the first and second jaws between the approximated and spaced apart configurations and a gimbal assembly configured to support the actuation member for off-axis movement and rotation.

### BRIEF DESCRIPTION OF THE DRAWINGS

The accompanying drawings, which are incorporated in and constitute a part of this specification, illustrate aspects of the disclosure and, together with a general description of the disclosure given above, and the detailed descriptions given below, serve to explain the principles of the disclosure, wherein:
FIG. 1 is a perspective view of a surgical grasper in accordance with the disclosure;
FIG. 2 is a top view of the surgical grasper of FIG. 1;
FIG. 3 is a side cross-sectional view of the surgical grasper of FIG. 1;
FIG. 4 is a partially enlarged cross-sectional view of the surgical grasper of FIG. 1, illustrating a handle assembly;
FIG. 5 is a partial perspective view of the surgical grasper of FIG. 1, illustrating first and second jaws in a spaced apart position; and
FIG. 6 is a side view of the surgical grasper of FIG. 1, illustrating movement of the handle assembly relative to an elongate shaft.

### DETAILED DESCRIPTION

The disclosure is described hereinbelow with reference to the accompanying drawings; however, it is to be understood that the disclosed graspers are merely exemplary of the disclosure and may be embodied in various forms. Well-known functions or constructions are not described in detail to avoid obscuring the disclosure in unnecessary detail. Therefore, specific structural and functional details disclosed herein are not to be interpreted as limiting, but merely as a basis for the claims and as a representative basis for teaching one skilled in the art to variously employ the disclosure in virtually any appropriately detailed structure. Like reference numerals refer to similar or identical elements throughout the description of the figures.

As used herein, the term "distal" refers to that portion of the instrument, or component thereof which is farther from the user while the term "proximal" refers to that portion of the instrument or component thereof which is closer to the user. As used herein, the term "about" means that the numerical value is approximate and small variations would not significantly affect the practice of the disclosed graspers. In addition, the terms parallel and perpendicular are understood to include relative configurations that are substantially parallel and substantially perpendicular up to about + or - 10 degrees from true parallel and true perpendicular.

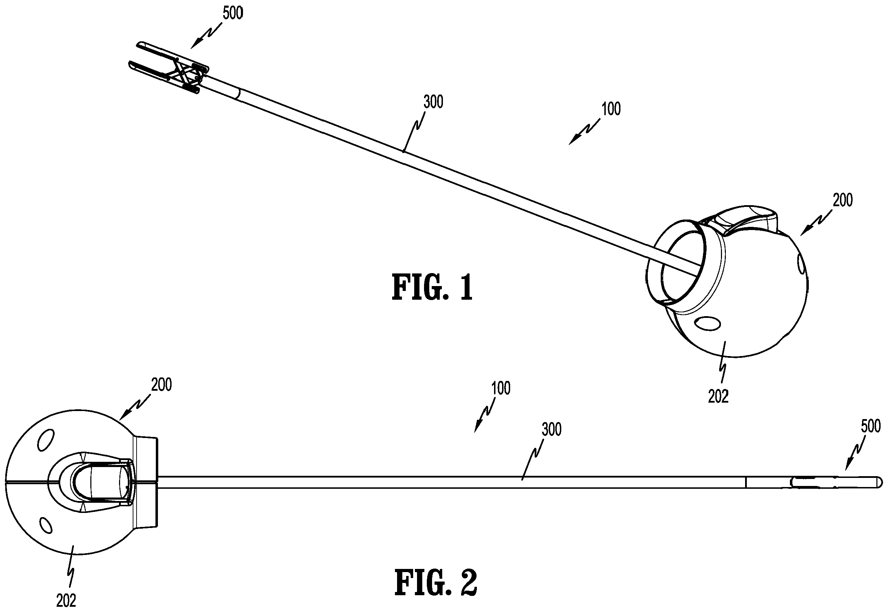

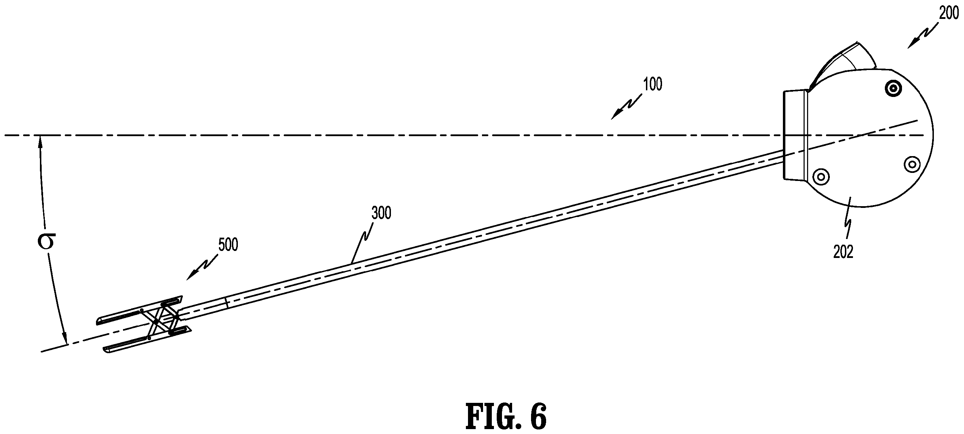

Referring to FIGS. 1 and 2, a surgical grasper is generally shown as a surgical grasper 100. The surgical grasper 100 includes a handle assembly 200, an elongate shaft 300 extending distally from the handle assembly 200, and a grasper assembly 500 operatively coupled to the handle assembly 200. The handle assembly 200 is configured to enable movement such as, e.g., off-axis rotation thereof, without actuating the grasper assembly 500. For example, the handle assembly 200 may be configured for off-axis movement defining an angle σ (FIG. 6) less than about 45 degrees with respect to the elongate shaft 300.

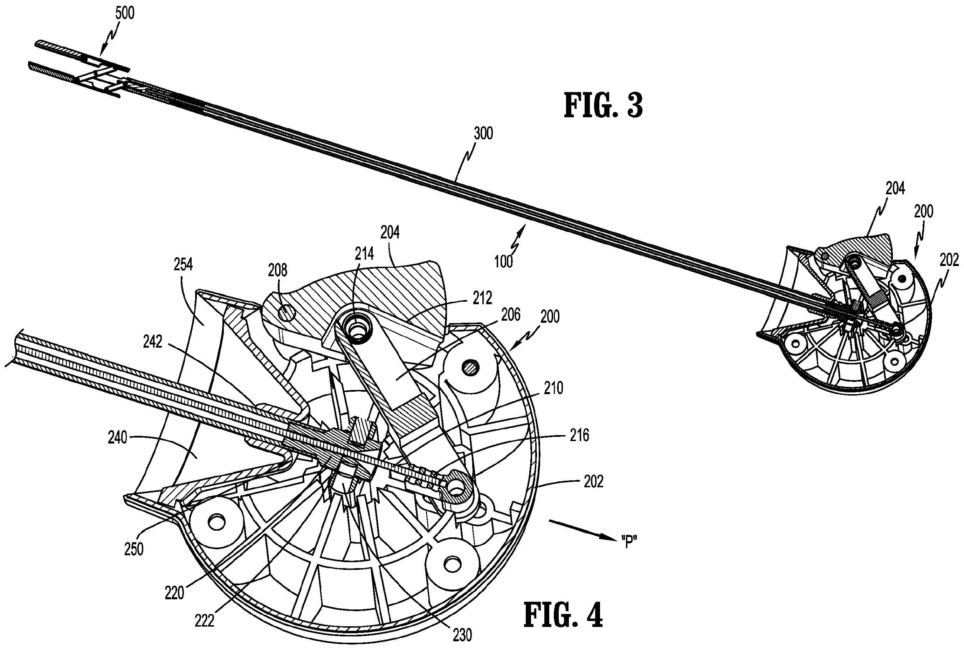

Under such a configuration, the clinician may hold the handle assembly 200 in a desired orientation most comfortable to the clinician to reduce fatigue during a surgical procedure. The handle assembly 200 is ergonomically designed to be held by a clinician. In particular, the handle assembly 200 has an arcuate profile. In particular, the handle assembly 200 includes a generally ball shaped body 202 for ambidextrous use. The ball shaped body 202 has a diameter in a range from about 1 inch and about 4 inches. In an aspect, the diameter may be about 2.4 inches. The ball shaped body 202 may be formed of a light biocompatible material such as, e.g., aluminum or polymetric materials, to reduce user fatigue. The ball shaped body 202 may define dimples (not shown) on an outer surface thereof to enhance gripping by the clinician. The ball shaped body 202 includes a gimbal assembly 250 (FIG. 4) to enable relative motion between the ball shaped body 202 and the elongate shaft 300 without imparting such movement to the grasper assembly 500.

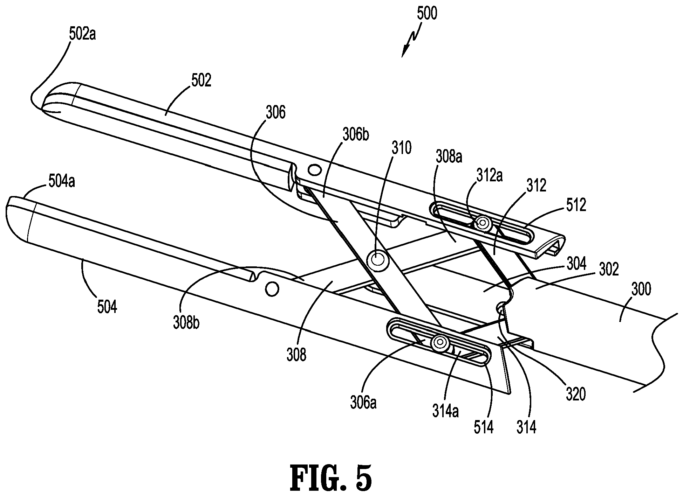

FIGS. 3 and 4 show the handle assembly 200 including an actuation button 204 at least partially extruding through the ball shaped body 202. The actuation button 204 is operatively coupled to the grasper assembly 500 to actuate the grasper assembly 500. The actuation button 204 is pivotably coupled to the ball shaped body 202 about a pivot 208. The actuation button 204 defines a recess 212 configured to support the linkage member 206 about a pivot 214. Under such a configuration, when the actuation button 204 is pressed by the clinician, the actuation button 204 urges the linkage member 206 to move proximally in the direction of an arrow "P", which, in turn, retracts an actuation member 210 proximally, thereby placing the first and second jaws 502, 504 in the approximated configuration. The actuation member 210 further includes a spring 216 to bias the actuation member 210 towards an advanced position prior to the actuation of the actuation button 204, thereby placing the first and second jaws 502, 504 in the spaced apart configuration. It is contemplated that a latch mechanism (not shown) may be operably coupled to the actuation button 204 and may include one or more mechanical interfaces configured to selectively engage a corresponding mechanical interface disposed within the ball shape body 202 of the handle assembly 200 to selectively lock the first and second jaws 502, 504 (FIG. 5) in the approximated or spaced apart configuration. In addition, the actuation member 210 may be formed of a flexible member to enable slight flexing of the actuation member 210 to accommodate movement of the handle assembly 200 without actuating of the grasper assembly 500.

As shown in FIG. 4, the ball shaped body 202 includes the gimbal assembly 250 having a support 220 movably supported by anchors 230, and a skirt 240. The support 220 defines a passage 222 configured to receive the actuation member 210 therethrough. The ball shaped body 202 defines an aperture 254. The aperture 254 is covered by the skirt 240 defining an opening 242 configured to receive the actuation member 210 via the elongate shaft 300 therethrough. The aperture 254 may define a conical shape. The surgical grasper 100 may be configured as a single-use device that is discarded after use or sent to a manufacturer for reprocessing, a reusable device capable of being cleaned and/or sterilized for repeated use by the end-user, or a partially-single-use, partially-reusable device. With respect to resuable, partially-single-use, partially-reusable configurations, the handle assembly 200 may be configured as a cleanable/sterilizable, reusable component, while the grasper assembly 500 and/or the elongate shaft 300 are configured as a single-use, disposable/reprocessable component. To this end, the skirt 240 may provide a fluid-tight seal against the elongate shaft 300. In addition, the skirt 240 may be formed of a flexible and/or resilient material such as, e.g., gel, foam, or an elastomer, to facilitate off-axis movement and/or rotation of the elongate shaft 300 relative to the ball shaped body 202 (FIG. 6). It is contemplated that the ball shaped body 202 may further include a locking mechanism (not shown) configured to selectively lock the position and/or orientation of the handle assembly 200 relative to the elongate shaft 300. It is further contemplated that the handle assembly 200 may further include a spring (not shown) to bias the handle assembly 200 towards a centered position, in which, the elongate shaft 300 is, e.g., concentrically, disposed with respect to the aperture 254 of the handle assembly 200.

FIG. 5 shows the grasper assembly 500 supported on a distal portion 302 of the elongate shaft 300. The grasper assembly 500 includes first and second jaws 502, 504 transitionable between an approximated configuration configured to grasp tissue therebetween and a spaced apart configuration in which, the first and second jaws 502 and 504 are spaced apart from each other and substantially parallel to each other. The first and second jaws 502, 504 may include respective tissue contacting surfaces 502a, 504a having a convex profile. Further, the first and second jaws 502, 504 having the convex profile may provide atraumatic contact with tissue grasped therebetween. In addition, the first and second jaws 502, 504 may include respective atraumatic tips. In particular, the distal portion 302 includes a support bar 304 supporting first and second linkages 306, 308 about a pivot 310. The support bar 304 defines a camming slot 320 configured to slidably receive a camming pin (not shown) pivotably coupled to third and fourth linkages 312, 314. Proximal ends of the third and fourth linages 312, 214 are pivotably connected to the actuation member 210 such that retraction of the actuation member 210 causes distal end portions 312a, 314a of the third and fourth linkages 312, 314 to move towards each other. The distal end portions 312a, 314a of the third and fourth linkages 312, 314 are pivotably coupled to the respective proximal end portions 306a, 308a of the first and second linkages 306, 308 such that retraction of the actuation member 210 causes the proximal end portions 306a, 308a of the first and second linkages 306, 308 towards each other. Distal end portions 306b, 308b of the first and second linkages 306, 308 are pivotably coupled to the respective first and second jaws 502, 504. Under such a configuration, actuation of the actuation button 204 causes retraction of the actuation member 210, which, in turn, transitions the first and jaws 502, 504 to the approximated position. In this manner, the first and second jaws 502, 504 provide more uniform pressure on tissue grasped therebetween compared to conventional jaws pivotably coupled about a single pivot.

While various graspers of the disclosure have been shown and described herein, it will be obvious to those skilled in the art that these graspers are provided by way of example only. Numerous variations, changes, and substitutions will now occur to those skilled in the art without departing from the disclosure. Accordingly, it is intended that the invention be limited only by the spirit and scope of the appended claims.

The invention may be described by reference to the following numbered paragraphs:-
1. A surgical grasper comprising:
   a tool assembly including first and second jaws transitionable between an approximated configuration, in which, the first and jaws are adjacent each other to grasp tissue therebetween and a spaced apart configuration, in which, the first and second jaws are spaced apart from each other;
   an elongate shaft supporting the tool assembly at a distal portion of the elongate shaft;
   an actuation member extending through the elongate shaft and operatively coupled to the tool assembly such that axial displacement of the actuation member causes transition of the first and second jaws between the approximated and spaced apart configurations; and
   a handle assembly having an arcuate body, the arcuate body including:
      an actuation button operatively coupled to the actuation member to transition the first and second jaws between the approximated and spaced apart configurations; and
      a gimbal assembly configured to support the elongate shaft and the actuation member for off-axis movement and rotation.
2. The surgical grasper according to paragraph 1, wherein the first and second jaws are biased towards the spaced apart configuration.
3. The surgical grasper according to paragraph 1, wherein the arcuate body of the handle assembly has a diameter in the range from about 1 inch and about 4 inches.
4. The surgical grasper according to paragraph 3, wherein the arcuate body of the handle assembly has a diameter of about 2.4 inches.
5. The surgical grasper according to paragraph 1, wherein the gimbal assembly is configured to maintain the configuration of the first and second jaws during off-axis movement and rotation of the handle assembly relative to the elongate shaft.
6. The surgical grasper according to paragraph 1, wherein the arcuate body is configured for off-axis movement less than about 45 degrees with respect to the elongate shaft.
7. The surgical grasper according to paragraph 1, wherein the arcuate body defines an aperture configured to receive the elongate shaft and the actuation member therethrough.
8. The surgical grasper according to paragraph 7, wherein the aperture of the arcuate body has a conical shape.
9. The surgical grasper according to paragraph 1, wherein the arcuate body includes a skirt configured to provide a fluid-tight seal against the elongate shaft.
10. The surgical grasper according to paragraph 1, wherein the arcuate body is spherical.
11. The surgical grasper according to paragraph 1, wherein the actuation member is at least partially flexible.
12. A surgical grasper comprising:
   a tool assembly including:
      first and second jaws transitionable between an approximated configuration, in which, the first and jaws are adjacent each other to grasp tissue therebetween and a spaced apart configuration, in which, the first and second jaws are spaced apart from each other; and
      first, second, third, and fourth linkage members, the first and second linkage members coupled to each other about a first pivot, the first and second linkage members coupled to the respective first and second jaws and the respective third and fourth linkage members about respective second and third pivots, the third and fourth linkage members pivotably coupled to each other about a fourth pivot;
   an elongate shaft supporting the tool assembly at a distal portion of the elongate shaft;
   an actuation member extending through the elongate shaft and operatively coupled to the tool assembly such that axial displacement of the actuation member causes transition of the first and second jaws between the approximated and spaced apart configurations; and
   a handle assembly including:
      an actuation button operatively coupled to the actuation member to transition the first and second jaws between the approximated and spaced apart configurations; and
      a gimbal assembly configured to support the elongate shaft and the actuation member for off-axis movement and rotation.
13. The surgical grasper according to paragraph 12, wherein the second and third pivots are movable along the respective first and second jaws.
14. The surgical grasper according to paragraph 12, wherein the fourth pivot is coupled to the actuation member for concomitant axial displacement therewith.
15. The surgical grasper according to paragraph 12, wherein the fourth pivot is movable along a groove defined in the elongate shaft.
16. The surgical grasper according to paragraph 12, wherein the first and second jaws include respective tissue contacting surfaces.
17. The surgical grasper according to paragraph 16, wherein each tissue contacting surface has a convex profile.
18. A surgical grasper comprising:
   a tool assembly including first and second jaws transitionable between approximated and spaced apart configurations;
   an actuation member operatively coupled to the tool assembly to transition the first and second jaws between the approximated and spaced apart configurations; and
   an arcuate body including:
      an actuation button operatively coupled to the actuation member to transition the first and second jaws between the approximated and spaced apart configurations; and
   a gimbal assembly configured to support the actuation member for off-axis movement and rotation.
19. The surgical grasper according to paragraph 18, wherein the arcuate body has a diameter in the range of about 1 inch and about 4 inches.
20. The surgical grasper according to paragraph 19, wherein the arcuate body has a diameter of about 2.4 inches.

## Claims

**1.** A surgical grasper comprising:
a tool assembly including first and second jaws transitionable between an approximated configuration, in which, the first and jaws are adjacent each other to grasp tissue therebetween and a spaced apart configuration, in which, the first and second jaws are spaced apart from each other;
an elongate shaft supporting the tool assembly at a distal portion of the elongate shaft;
an actuation member extending through the elongate shaft and operatively coupled to the tool assembly such that axial displacement of the actuation member causes transition of the first and second jaws between the approximated and spaced apart configurations; and
a handle assembly having an arcuate body, the arcuate body including:
an actuation button operatively coupled to the actuation member to transition the first and second jaws between the approximated and spaced apart configurations; and
a gimbal assembly configured to support the elongate shaft and the actuation member for off-axis movement and rotation.

**2.** The surgical grasper according to claim 1, wherein the first and second jaws are biased towards the spaced apart configuration.

**3.** The surgical grasper according to claim 1 or claim 2, wherein the arcuate body of the handle assembly has a diameter in the range from about 1 inch and about 4 inches; preferably wherein the arcuate body of the handle assembly has a diameter of about 2.4 inches.

**5.** The surgical grasper according to any preceding claim, wherein the gimbal assembly is configured to maintain the configuration of the first and second jaws during off-axis movement and rotation of the handle assembly relative to the elongate shaft.

**6.** The surgical grasper according to any preceding claim, wherein the arcuate body is configured for off-axis movement less than about 45 degrees with respect to the elongate shaft.

**7.** The surgical grasper according to any preceding claim, wherein the arcuate body defines an aperture configured to receive the elongate shaft and the actuation member therethrough.; preferably wherein the aperture of the arcuate body has a conical shape.

**8.** The surgical grasper according to any preceding claim, wherein the arcuate body includes a skirt configured to provide a fluid-tight seal against the elongate shaft; preferably wherein the arcuate body is spherical.

**9.** The surgical grasper according to any preceding claim, wherein the actuation member is at least partially flexible.

**10.** A surgical grasper comprising:
a tool assembly including:
first and second jaws transitionable between an approximated configuration, in which, the first and jaws are adjacent each other to grasp tissue therebetween and a spaced apart configuration, in which, the first and second jaws are spaced apart from each other; and
first, second, third, and fourth linkage members, the first and second linkage members coupled to each other about a first pivot, the first and second linkage members coupled to the respective first and second jaws and the respective third and fourth linkage members about respective second and third pivots, the third and fourth linkage members pivotably coupled to each other about a fourth pivot;
an elongate shaft supporting the tool assembly at a distal portion of the elongate shaft;
an actuation member extending through the elongate shaft and operatively coupled to the tool assembly such that axial displacement of the actuation member causes transition of the first and second jaws between the approximated and spaced apart configurations; and
a handle assembly including:
an actuation button operatively coupled to the actuation member to transition the first and second jaws between the approximated and spaced apart configurations; and
a gimbal assembly configured to support the elongate shaft and the actuation member for off-axis movement and rotation, preferably wherein the second and third pivots are movable along the respective first and second jaws.

**11.** The surgical grasper according to claim 10, wherein the fourth pivot is coupled to the actuation member for concomitant axial displacement therewith.

**12.** The surgical grasper according to claim 10 or claim 11, wherein the fourth pivot is movable along a groove defined in the elongate shaft; and/or wherein the first and second jaws include respective tissue contacting surfaces; preferably wherein each tissue contacting surface has a convex profile.

**13.** A surgical grasper comprising:
a tool assembly including first and second jaws transitionable between approximated and spaced apart configurations;
an actuation member operatively coupled to the tool assembly to transition the first and second jaws between the approximated and spaced apart configurations; and
an arcuate body including:
an actuation button operatively coupled to the actuation member to transition the first and second jaws between the approximated and spaced apart configurations; and
a gimbal assembly configured to support the actuation member for off-axis movement and rotation.

**14.** The surgical grasper according to claim 13, wherein the arcuate body has a diameter in the range of about 1 inch and about 4 inches.

**15.** The surgical grasper according to claim 14, wherein the arcuate body has a diameter of about 2.4 inches.
